(19) Europäisches Patentamt
European Patent Office
Office européen des brevets

(11) Publication number: **0 241 232 B1**

(12) **EUROPEAN PATENT SPECIFICATION**

(45) Date of publication of patent specification: **27.02.91 Bulletin 91/09**

(51) Int. Cl.$^5$: **C07D 413/06**, A01N 43/26, A61K 31/42

(21) Application number: **87302901.1**

(22) Date of filing: **02.04.87**

(54) **Isoxazolylethanol derivatives.**

The file contains technical information submitted after the application was filed and not included in this specification

(30) Priority: **03.04.86 JP 77434/86**

(43) Date of publication of application: **14.10.87 Bulletin 87/42**

(45) Publication of the grant of the patent: **27.02.91 Bulletin 91/09**

(84) Designated Contracting States: **AT BE CH DE ES FR GB GR IT LI LU NL SE**

(56) References cited:
**EP-A- 0 099 165**
**EP-A- 0 110 048**
**EP-A- 0 141 205**
**EP-A- 0 158 205**
**CHEMICAL ABSTRACTS, vol. 85, no. 11, September 13, 1976, Columbus, Ohio, USA; HAMASHIMA, YOSHIO; MINAMI; KYOJI "3-(1,2-Epoxyethyl)- and 3-(1-hydroxy-2-aminoethyl)-5-phenylisoxazoles." page 545, column 1, abstract no. 78107q & Japan. Kokai 75,131,965 18 October 1975**
**CHEMICAL ABSTRACTS, vol. 81, no. 23, December 9, 1974, Columbus, Ohio, USA; KANO, HIDEO; TAKAHASHI, SHIRO "Isoxazole derivates." page 534, column 2, abstract no. 152209k & Japan. Kokai 74 41,272 18 May 1974**

(56) References cited:
**CHEMICAL ABSTRACTS; vol. 82, no. 1, January 6, 1975, Columbus, Ohio, USA; KANO, HIDEO; TAKAHASHI, SHIRO "3-(2-Cyclic amino-1-hydroxyethyl)-5-(hydroxyphenyl) -isoxazoles.) page 368, column 1, abstract no. 4232t & Japan. Kokai 74 54,366 27 May 1974**
**CHEMICAL ABSTRACTS, vol. 82, no. 1, January 6, 1975, Columbus, Ohio, USA; KANO, HIDEO; TAKAHASHI, SHIRO "3-(w-Cyclic amino-alpha-hydroxyalkyl)-5-(hydroxyphenyl)-isoxazoles." page 368, column 1, abstract no. 4234v & Japan. Kokai 74 54,365 27 May 1974**

(73) Proprietor: **SHIONOGI SEIYAKU KABUSHIKI KAISHA**
**12, Dosho-machi, 3-chome Higashi-ku, Osaka-shi**
**Osaka 541 (JP)**

(72) Inventor: **Makisumi, Yasuo**
**2-5-17, Midoridai**
**Kawanishi-shi Hyogo (JP)**
Inventor: **Murabayashi, Akira**
**13-31, Hoshimi-cho**
**Ibaraki-shi Osaka (JP)**
Inventor: **Tawara, Katsuya**
**1-1-814, Higashiota**
**Ibaraki-shi Osaka (JP)**
Inventor: **Hatta, Takayuki**
**116-38, Ohazasagami§Koga-cho**
**Koga-gun Shiga (JP)**

(74) Representative: **Hardisty, David Robert et al**
**BOULT, WADE & TENNANT 27 Furnival Street**
**London EC4A IPQ (GB)**

EP 0 241 232 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to isoxazolylethanol derivatives. More particularly, this invention is directed to isoxazolylethanol derivatives which have been found to be particularly useful as antimycotic agents and/or agricultural fungicides, to their production, to their use and to pharmaceutical or agricultural formulations containing the compounds.

Azolylethanol derivatives containing a heterocyclic ring other than the azolyl ring within the molecule have been disclosed to be useful as antimycotics or agricultural fungicides in European Patents Nos 46,337, 102,727 and 158,205. However, some of these compounds are unfortunately accompanied by strong adverse actions such as hepatic toxicity or tetratogenecity, depending upon the sort of the compounds.

According to the present invention, there is provided an isoxazolylethanol derivative of the formula :

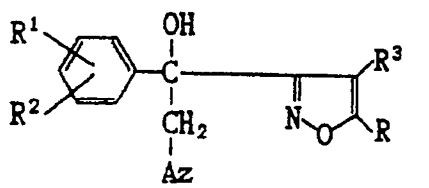

(I)

(wherein Az is imidazolyl or triazolyl ;

R is hydrogen or $C_1$-$C_5$ alkyl ;

$R^1$, $R^2$ and $R^3$ are each independently hydrogen or halogen), the compound optionally being as a salt thereof.

The antimycotic and/or fungicidal compounds (I) of the present invention may be used for human or veterinary medicines or fungicides. Accordingly the invention also provides a pharmaceutical or veterinary formulation comprising as an active ingredient at least one antimycotic compound of the formula (I) formulated for pharmaceutical or veterinary use, respectively, and generally associated with one or more pharmaceutically acceptable carriers, diluents and/or excipients, in which case the active ingredient is usually in an amount of from 0.1 to 95% by weight. The pharmaceutical or veterinary formulations may be in unit dosage form.

Further included are agricultural fungicidal formulations comprising a fungicidal compound of formula I formulated for agricultural use. As used herein, "agriculture" includes horticulture.

This invention further provides a process for preparing the compound (I) as shown in the following scheme :

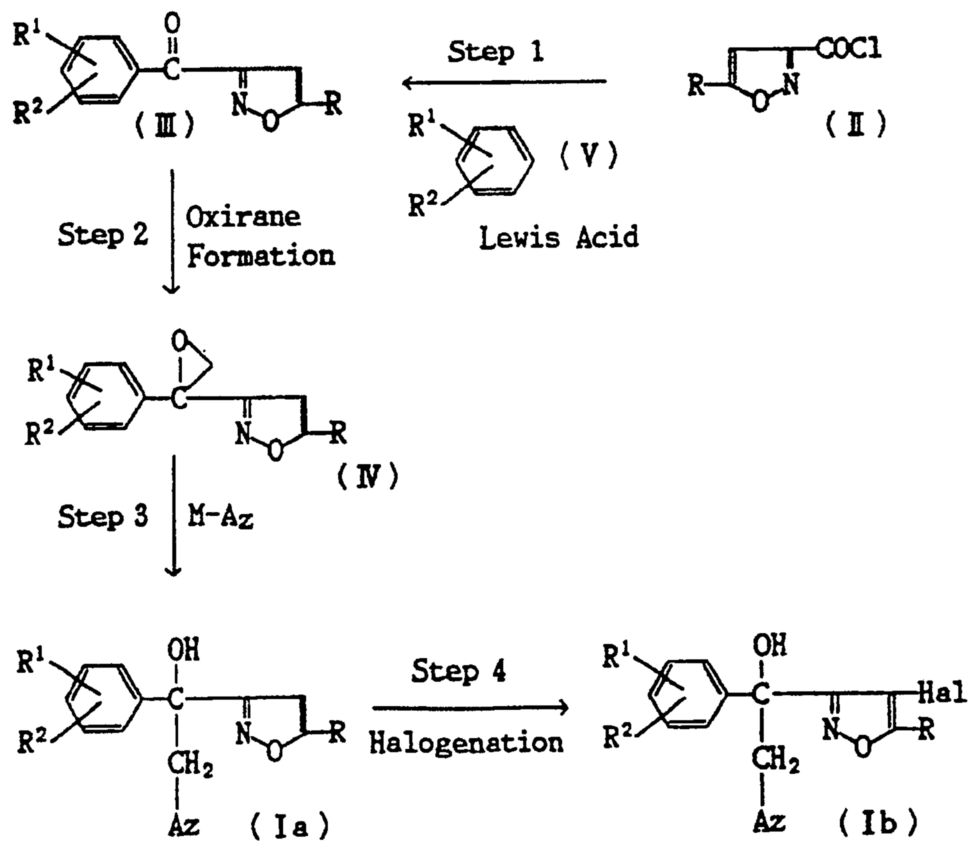


(wherein M is hydrogen or an alkali metal atom, and Az, R, $R^1$, $R^2$ and $R^3$ are each as defined above).

The process is described further below by way of example with reference to non limiting exemplary reaction processes and conditions.

Step 1

The starting acid chloride (II) is allowed to react with the optionally substituted benzene (V) in the presence of Lewis acid such as aluminum chloride, zinc chloride or the like at a temperature of 0 to 100°C, preferably 50 to 90°C, whereby the optionally substituted benzoylisoxazole (III) is obtained.

The starting material (II) can be prepared in a conventional manner, via an isoxazole ester obtainable by the method as disclosed in T.S. Gardner et al., J. Org. Chem., 26, 1514 (1961).

Step 2

The ketone (III) obtained above is subjected to the oxirane formation, whereby the ethylene oxide (IV) is obtained. The oxirane formation may be the E.J. Corey*1 method using trimethylsulfoxonium iodide in the presence of sodium hydride in dimethyl sulfoxide or another method*2 using trimethylsulfoxonium iodide in the presence of a phase transfer catalyst.

*1 E.J. Corey, J. Am. Chem. Soc., 87, 1353 (1965)

*2 E.J. Corey, J. Am. Chem. Soc., 97, 1626 (1975)

Step 3

The starting oxirane (IV) is allowed to react with an alkali metal salt of azole such as 1H-1, 2, 4-triazole or 1H-imidazole. The alkali metal salt includes sodium and potassium salts. The reaction is performed in a solvent, e.g. dimethylformamide at a temperature of, for example, 20 to 100°C. Thus the product (Ia) is obtained.

Step 4

The product (Ia) obtained above is subjected to halogenation such as fluorination, chlorination, bromination or iodination in a conventional manner, whereby the corresponding halo compound (Ib) is obtained.

The objective compound (I) can be converted into pharmaceutically, veterinarily or agriculturally acceptable acid addition salts thereof. Examples of such acids are an organic acid such as citric acid, malic acid, succinic acid, oxalic acid or methanesulfonic acid and an inorganic acid such as hydrohalogenic acid, nitric acid, sulfuric acid or phosphoric acid.

The antimycotic compounds (I) or their salts are highly valuable as oral or parenteral antimycotics, showing excellent antimycotic activity. Further they are also useful as veterinary antimycotics, agricultural fungicides or fungicidal coating additives or for other applications to inanimate subjects. For clinical application, the compounds (I) or their salts are generally applicable alone or in combination with one or more pharmaceutically acceptable carriers, diluents, and/or excipients selected depending upon administering route and pharmaceutically standard practice. For example, they may be used in the form of tablets or capsules containing one or more disintegrators such as starch or lactose or in the form of a suspension. For parenteral application, they may be used in intravenous, intramuscular, or subcutaneous route in the form of sterile aqueous solution containing other additives (for example, glucose, sodium chloride in an amount enough to make the injection isotonic with blood, or the like). Appropriate daily dosage of the compound (I) or its salts is 5 to 500 mg., preferably 10 to 250 mg for oral use and 0.5 to 20 mg for the parenteral route. However, the dosage can be changed depending upon the age, sex end conditions of the patient.

The fungicidal compounds (I) or their acid addition salts show potent fungicidal activities against various pathogenic fungi and other soil-born pathogens such as powdery mildew, downy mildew, sheath blight, damping-off or the like in vegetables, fruit plants, rice plants, wheat or other plants.

The compounds (I) or their salts may be applied in a formulation suitable for agricultural fungicides such as emulsions, solutions, wettable powders, dusts, suspensions, granules, aerosols, smokes, pastes or the like. They may be used alone or in combination with one or more solid or liquid carriers, diluents or excipients. Representative solid carriers are clay, talc, diatomaceous earth, silica, kaolin, bentonite, pumice and the like. Exemplary liquid carriers include water, methanol, ethanol, ethylene glycol, dimethylformamide, dimethyl sulfoxide, acetone, methyl ethyl ketone, cellosolve, dioxane, diglyme and the like. When desired, there may be added appropriate adjuvants such as emulsifiers, dispersants, spreaders, surfactants, wetting agents, stabilizers, synergists or the like. Moreover, the compounds (I) or their salts may be used in combination with other pesticides such as fungicides, germicides, insecticides, herbicides, repellents, miticides, nematocides, plant growth regulators or the like. Appropriate application rate of the compounds (I) or their salt is in the range of about 10 to 500 ppm and about 2 to 200 g/10 are. Test compounds can be applied by foliar, soil-drench, soil-mix or seed treatment. For seed application, seeds may be soaked or dressed. The concentration or volume of the compound can be selected, depending upon plant species, development stage of plant, or way of application. For example, the compound can be sprayed foliarly in 0.1 to 1000 ppm, drenched into soil with 10 to 2000 times diluents, soaked into seeds with 10 to 100 times diluents, or dressed seeds with 0.1 to 5% (w/w) per seed using various formulations containing 0.5 to 99.9% (w/w) active ingredient.

Presently preferred and practical embodiment of the present invention ar illustratively shown in the follovning examples.

Example 1

3-(4-Chlorobenzoyl) isoxazole

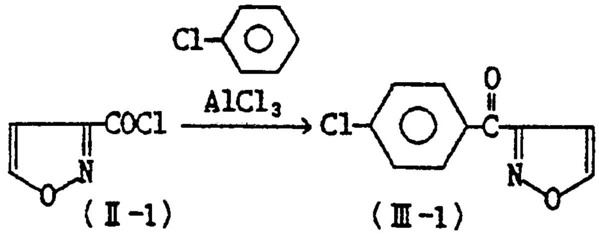


(II-1) (III-1)

To 3 g of 3-isoxazolecarboxylic acid were added 4 ml of thionyl chloride and 0.1 ml of pyridine. The

mixture was refluxed for 2 hr. and concentrated under reduced pressure. To the residue were added 6 ml of chlorobenzene and 4.24 g of aluminium chloride. The mixture was heated for 2 hr. at 90°C under stirring. The reaction mixture was mixed with water and extracted with benzene. The organic layer was washed with 5% aqueous potassium carbonate and water, dried over anhydrous sodium sulfate and concentrated. The residue was recrystallized from ether - hexane to give 3.11 g of the titled compound.

m.p. : 55 - 55.5 °C

Yield : 56.5 %

$\nu_{max}^{Nujol}$ : 1662 $cm^{-1}$

Example 2-12

The reaction was performed as in Example 1, whereby the ketones (III) as shown in table 1 were obtained.

Table 1

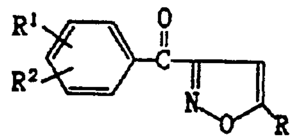

(III)

| Ex. No. | R | $R^1$ | $R^2$ | m.p. (°C) | IR, ν Solvent | IR, ν $cm^{-1}$ | Compd. No. |
|---|---|---|---|---|---|---|---|
| 2 | H | H | 4-F | 77 - 78 | Nujol | 1650 | III- 2 |
| 3 | H | 2-Cl | 4-Cl | 103 - 104 | Nujol | 1681 | III- 3 |
| 4 | H | 2-F | 4-F | 72 - 73 | Nujol | 1680 | III- 4 |
| 5 | $CH_3$ | H | 4-Cl | 66 - 67 | Nujol | 1662 | III- 5 |
| 6 | $CH_3$ | H | 4-F | 68 - 69 | Nujol | 1670 | III- 6 |
| 7 | $CH_3$ | 2-Cl | 4-Cl | 121 - 122 | Nujol | 1674 | III- 7 |
| 8 | $CH_3$ | 2-F | 4-F | 97 - 98 | Nujol | 1684 | III- 8 |
| 9 | $CH_3$ | H | H | oily substance | Chloroform | 1658 | III- 9 |
| 10 | Et | 2-F | 4-F | oily substance | — | — | III- 10 |
| 11 | n-Bu | 2-F | 4-F | oily substance | Film | 1678 | III- 11 |
| 12 | t-Bu | 2-F | 4-F | oily substance | Film | 1672 | III- 12 |

Example 13

1-(4-Chlorophenyl)-1-(isoxazol-3-yl)oxirane

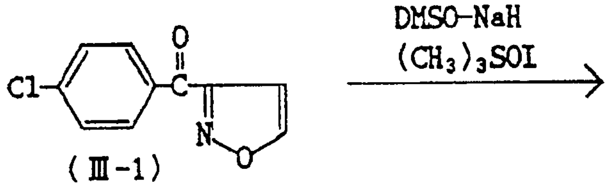


(Ⅲ-1)

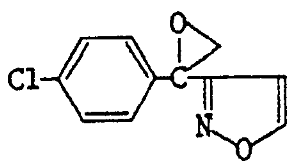

(Ⅳ-1)

To a solution of 40 ml of dimethyl sulfoxide was added 60% sodium hydride (mineral oil suspension : 717 mg), and the mixture was heated for 1.5 hr. at 70°C. To the mixture was added 4.02 g of trimethylsulfoxonium iodide under ice-cooling and stirring and the reaction mixture was stirred for 5 min. A solution of 3.10 g of 3-(4-chlorobenzoyl)isoxazole dissolved in 25 ml of tetrahydrofuran was added dropwise to the mixture, and the resultant mixture was stirred for 15 hr. at room temperature and concentrated. Water was added to the reaction mixture, and the solution was extracted with benzene, washed with water, dried over anhydrous sodium sulfate and concentrated. The residue was subjected to silica gel column chromatography eluting with benzene. Evaporation of the eluate gave 2.70 g of the titled compound as an oily substance.

Yield : 81.5%

Example 14-24

The reaction was performed as in Example 13, whereby oxirane (IV) as shown in Table 2 were obtained.

Table 2

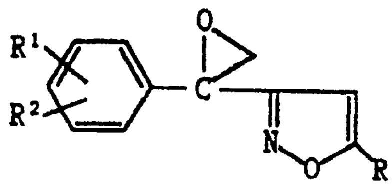

(IV)

| Ex. No. | R | $R^1$ | $R^2$ | m.p. (°C) | Yield (%) | Compd. No. |
|---|---|---|---|---|---|---|
| 14 | H | H | 4-F | oily substance | 79 | IV - 2 |
| 15 | H | 2-Cl | 4-Cl | 95 - 96 | 63 | IV - 3 |
| 16 | H | 2-F | 4-F | oily substance | 56 | IV - 4 |
| 17 | $CH_3$ | H | 4-Cl | oily substance | 87 | IV - 5 |
| 18 | $CH_3$ | H | 4-F | oily substance | 83 | IV - 6 |
| 19 | $CH_3$ | 2-Cl | 4-Cl | 86 - 87 | 81 | IV - 7 |
| 20 | $CH_3$ | 2-F | 4-F | 33 - 34 | 69 | IV - 8 |
| 21 | $CH_3$ | H | H | oily substance | 75 | IV - 9 |
| 22 | Et | 2-F | 4-F | oily substance | 60 | IV - 10 |
| 23 | n-Bu | 2-F | 4-F | oily substance | 95 | IV - 11 |
| 24 | t-Bu | 2-F | 4-F | oily substance | 96 | IV - 12 |

Example 25

1-(4-Fluorophenyl)-1-(isoxazol-3-yl)-2-(1H-1,2,4-triazol-1-yl)ethanol

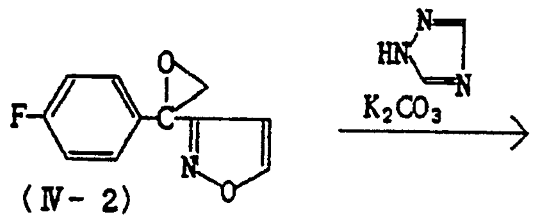


(IV- 2)

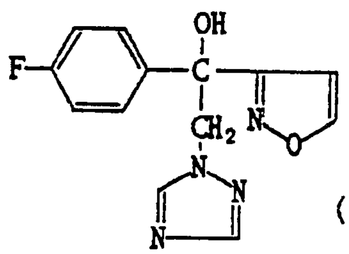

(Ia-1)

To a solution of 2.53 g of 1-(4-fluorophenyl)-1-(isoxazol-3-yl)oxirane dissolved in 20 ml of dimethylformamide were added 1.68 g of 1H-1,2,4-triazole and 5.0 g of potassium carbonate, and the mixture was heated for 3 hr. at 60°C under stirring. After cooling, the reaction solution was filtered, and the filtrate was concentrated. The residue was added with water, and the mixture was extracted with benzene, washed with water, dried and concentrated. The residue was subjected to silica gel column chromatography, eluting with chlorofolm - methanol (100 : 1 v/v). Evaporation of the eluate gave 1.87 g of the crystal melting at 132-134°C. The crystal was recrystallized from acetone -hexane to give 1.46 g of the titled compound.

m.p. : 133 - 134 °C

Yield : 43.8 %

Anal Calcd. (%) for $C_{13}H_{11}N_4O_2F$ :

C : 56.93; H : 4.04; N : 20.43; F : 6.93

Found (%) : C : 57.08; H : 4.28; N : 19.99; F : 6.88

$H^1$-NMR : $\delta^{CDCl_3}$ 4.62 (1H, d, 15Hz), 5.05 (1H, d, 15Hz), 5.83 (1H)

Example 26-36

The reaction was performed as in Example 25, whereby the compounds (Ia) as snown in Table 3 were obtained.

Table 3

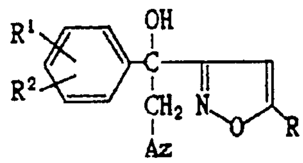

( I a )

where Az=1H-1,2,4-triazol-1-yl

| Ex. No. | R | $R^1$ | $R^2$ | m.p. | Yield (%) | NMR $\delta_{CDCl_3}$ 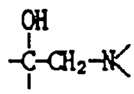 | Compd. No. |
|---|---|---|---|---|---|---|---|
| 26 | H | H | 4-Cl | 134 - 135 | 37 | 4.26d (J=14Hz)<br>5.03d (J=14Hz) | I a - 2 |
| 27 | H | 2-Cl | 4-Cl | 180 - 181 | 15 | 5.25d (J=15Hz)<br>5.49d (J=15Hz) | I a - 3 |
| 28 | H | 2-F | 4-F | 158 - 159 | 41 | 5.13s | I a - 4 |
| 29 | $CH_3$ | H | 4-Cl | 137 - 138 | 50 | 4.59d (J=15Hz)<br>5.00d (J=15Hz) | I a - 5 |
| 30 | $CH_3$ | H | 4-F | 104 - 105 | 41 | 4.56d (J=13Hz)<br>5.01d (J=13Hz) | I a - 6 |
| 31 | $CH_3$ | 2-Cl | 4-Cl | 184 - 185 | 86 | 5.15d (J=15Hz)<br>5.45d (J=15Hz) | I a - 7 |
| 32 | $CH_3$ | 2-F | 4-F | 166 - 167 | 58 | 5.12s | I a - 8 |
| 33 | $CH_3$ | H | H | 156 - 157 | 46 | 4.62d (J=14Hz)<br>5.02d (J=14Hz) | I a - 9 |
| 34 | Et | 2-F | 4-F | 133 - 134 | 47 | 5.11s | I a - 10 |
| 35 | n-Bu | 2-F | 4-F | 123 - 124 | 90 | 5.10s | I a - 11 |
| 36 | t-Bu | 2-F | 4-F | 171 - 172 | 72 | 5.02d (J=13Hz)<br>5.21d (J=13Hz) | I a - 12 |

Example 37

1-(4-Fluorophenyl)-1-(5-methylisoxazol-3-yl)-2-(1H-imidazol-1-yl)ethanol

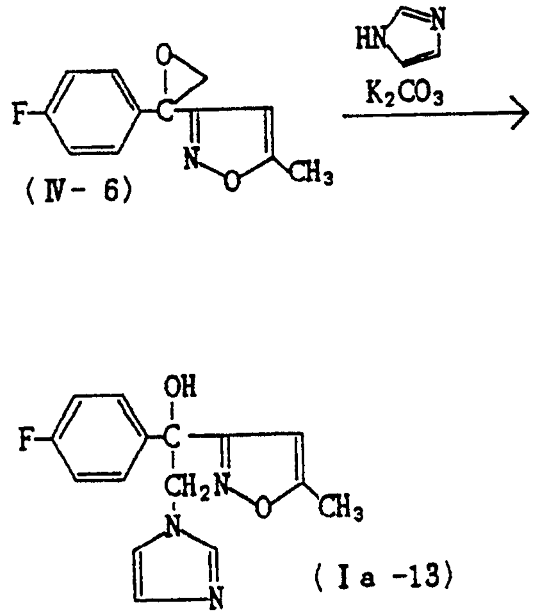


(Ⅳ- 6)

(Ⅰa -13)

A mixture of 357 mg of 1-(4-fluorophenyl)-1-(5-methylisoxazol-3-yl)oxirane, 6 ml of dimethylformamide, 333 mg of 1H-imidazole and 645 mg of potassium carbonate were heated for 8 hr. at 90°C under stirring. Dimethylformamide was distilled off under reduced pressure. The mixture was mixed with water and extracted with benzene. The benzene layer was washed with water, dried over anhydrous sodium sulfate and concentrated. The residue was subjected to silica gel column chromatography (10 g of silica gel), the fraction of chloroform-methanol (50 : 1 v/v) gave 158 mg of the title compound. The compound was recrystallized from acetone- hexane to give 115 mg of the crystal.

m.p. : 182 - 183 °C

Anal Calcd. (%) for $C_{15}H_{14}N_3O_2F$ :

C: 62.71; H: 4.91; N: 14.63; F: 6.61

Found (%) : C: 62.36; H: 4.86; N: 14.50; F: 6.78

Example 38

1-(4-Chlorophenyl)-1-(5-methyl-4-chloroisoxazol-3-yl)-2-(1H-1,2,4-triazol-1-yl)ethanol

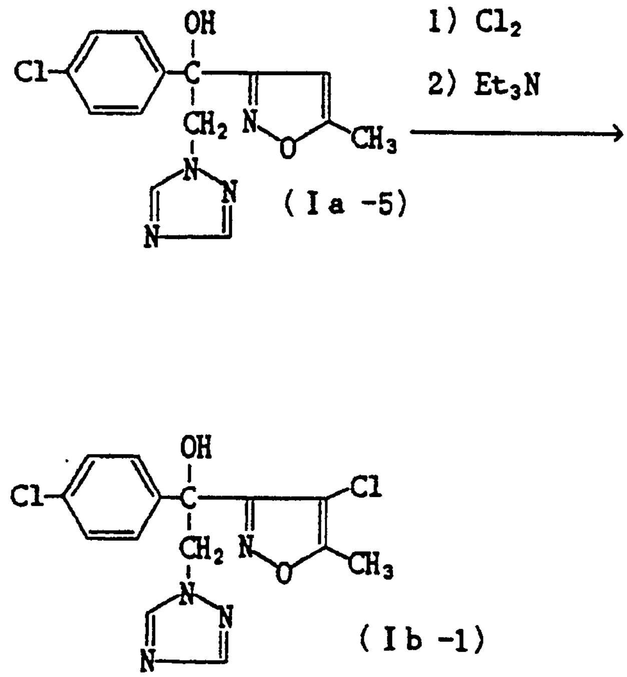


(Ia -5)

(Ib -1)

To a solution of 200 mg of 1-(4-chlorophenyl)-1-(5-methylisoxazol-3-yl)-2-(1H-1,2,4-triazol-1-yl) ethanol in 10 ml of methylene chloride was added a solution of 70 mg of chlorine in carbon tetrachloride at room temperature under stirring, and the mixture was allowed to stand overnight at room temperature. The solution was mixed with 1.5 ml of triethylamine, stirred for an hour at room temperature and concentrated. The residue was mixed with 5% aqueous potassium carbonate solution and extracted with benzene. The benzene layer was washed with water, dried over anhydrous sodium sulfate and concentrated. The residue was recrystallized from ether - hexane to give 190 mg of the titled compound.

m.p. : 162 - 164 °C

Yield : 85 %

Anal Calcd. (%) for $C_{14}H_{12}N_4O_2Cl$ :

C : 49.58; H : 3.57; N : 16.52; Cl : 20.90

Found (%) : C : 49.83; H : 3.61; N : 16.42; Cl : 20.89

Example 39-40

The reaction was performed as in Example 38, whereby the chlorides (Ib) as shown in Table 4 were obtained.

Table 4

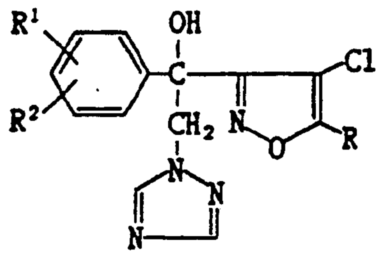

(I b)

| Ex. No. | R | $R^1$ | $R^2$ | m.p. (°C) | Yield (%) | Compd. No. |
|---|---|---|---|---|---|---|
| 39 | $CH_3$ | H | 4-F | 152 - 153 | 78 | I b -2 |
| 40 | $CH_3$ | H | H | 111 - 113 | 90 | I b -3 |

Pharmaceutical Formulation

(1) 1-(4-Fluorophenyl)-1-(isoxazol-3-yl)-2-(1H-1,2,4-triazol-1-yl)ethanol I a -1 ........ 25 mg

Lactose ........ 100 mg

Wheat starch ........ 15 mg

Gelatin ........ 5 mg

Magnesium stearate ........ 5 mg

Total 150 mg

The above components were mixed and charged in a capsule to prepare a capsule.

## Agricultural Formulations

(1) 1-(4-Fluorophenyl)-1-(isoxazol-3-yl)-2-(1H-1,2,4-triazol-1-yl)ethanol Ia-1 ........ 5 parts
Propyl alcohol ........ 20 parts
Polyoxyethylene alkylphenyl ether ........ 5 parts
Water ........70 parts

Total 100 parts

The above components were mixed each other to prepare water soluble powders.

(2) 1-(4-Fluorophenyl)-1-(5-methylisoxazol-3-yl)-2-(1H-imidazol-1-yl)ethanol Ia-13 ........ 50 parts
Sodium alkylbenzenesulfonate ........ 6 parts
Sodium ligninsulfonate ........ 4 parts
Clay ........ 40 parts

Total 100 parts

The above components were mixed and pulverized to prepare a wettable powder.

(3) 1-(4-Fluorophenyl)-1-(5-methylisoxazol-3-yl)-2-(1H-imidazol-1-yl)ethanol Ia-13 ........ 5 parts
Mixture of bentonite and talc (1:1 w/w) ........ 90 parts
Sodium alkylbenzenesulfonate ........ 5 parts

Total 100 parts

The above components were mixed, pulverized and subjected to a granulator to give granules.

(4) 1-(4-Chlorophenyl)-1-(5-methylisoxazol-3-yl)-2-(1H-1,2,4-triazol-1-yl)ethanol Ib-1 ........ 25 parts
Polyoxyethylenealkylphenyl ether ........ 8 parts
Sodium alkylbenzenesulfonate ........ 2 parts
Xylene ........ 65 parts

Total 100 parts

The above components were mixed each other to prepare an emulsion.

| | | |
|---|---|---|
| (5) 1-(4-Chlorophenyl)-1-(5-methylisoxazol-3-yl)-2-(1H-1,2,4-triazol-1-yl)ethanol Ib-1 | ········ | 1 parts |
| Talc | ········ | 99 parts |
| | Total | 100 parts |

The above components were mixed and made into powder. In the above formulations the parts are by weight.

Effect of the Invention

Experiment 1

Inhibition test against pseudomycelium formation of Candida albicans

Candida albicans KE-2 was inoculated to Eagle-MEM medium (Nissan 2 made by Nissui Seiyaku, Ltd.) supplemented with 20% calf serum so that its final inoculum of yeast cell might be $1 \times 10^6$ yeast cells per milliliter. The test compounds were added thereon in two fold dilution series. After incubation for 18 hours at 37°C, the fungal growth in each drug concentration was smeared and fixed on a slide glass, and the presence or absence of the pseudomycelium formation was examined by a microscope following Giemsa staining. Minimum effective concentration was defined as the lowest concentration of the compound which prevented typical pseudomycelium formation.

The results are shown in Table 5.

Result

Table 5

| Compd. No. | Inhibitory Concentration of Pseudo-myselium Formation ( μg/ml ) |
|---|---|
| I a - 1 | 0.31 |
| I a - 2 | 0.04 |
| I a - 3 | 0.02 |
| I a - 4 | 0.16 |
| I a - 5 | 0.02 |
| I a - 6 | 0.16 |
| I a - 7 | 0.02 |
| I a - 8 | 0.08 |
| I a - 9 | 0.31 |
| I a - 10 | 0.08 |
| I a - 11 | 0.04 |
| I a - 12 | 0.31 |
| I a - 13 | 0.63 |
| I b - 1 | 0.31 |
| I b - 2 | 1.25 |
| I b - 3 | 1.25 |

Experiment 2

Therapeutic effect against experimental candidiasis in mice

After Candida albicans KE-2 was incubated on Sabouraud's glucose agar at 28°C for 48 hr., it was suspended in Sabouraud's glucose broth and the cells ($2 \times 10^6$) were injected to a tail vein of Jcl-ICR female mouse (4 weeks age ; weight : 18-20 g). A suspension of the test compound in 2% gum arabic was orally administered three times, immediately after infection of the yeast (0 hour), after 4 hours and after 24 hours, as a dosage of 50 mg/kg. Non-treated group was used as control. Each eight mice per group was used in control group and treated group. All the mice in control group died within 48 hr. after infection.

Therapeutic effect of the test compound was judged by mouse survival ratio on the 5th day after infection.

The result are shown in Table 6.

Result

Table 6

| Compd. No. | Therapeutic Effect (%) |
|---|---|
| Ia - 1 | 87.5 |
| Ia - 2 | 100 |
| Ia - 3 | 100 |
| Ia - 4 | 100 |
| Ia - 5 | 87.5 |
| Ia - 8 | 50 |
| Ia - 10 | 50 |
| Ia - 11 | 50 |

Experiment 3

Control Activities by Seed-Soaking to Crown Rust in Oats, Powdery Mildews in Cucumber and Wheat, and Rhizoctonia Damping-off in Cucumber

Methods

Application of Test Compound :

The test compound was dissolved in 0.2 ml of acetone and suspended in demineralized distilled water containing a small amount of spreader, to a concentration of 500 ppm. The suspension was further diluted to one quarter dilution with demineralized distilled water (containing a small amount of spreader) to give a suspension in 125 ppm. All seeds of oats, wheat and cucumber were soaked in these suspensions at room temperature for an hour and then the seeds were air-dried.

Inoculation of Pathogens

The seeds as described above were sown in plastic pots (diameter : 9 cm). For rust and powdery mildews, the pots were kept in a glasshouse for 1-3 weeks until the seeds grew to young plants. The young oats (10-20 plants per pot) were inoculated by dusting with spores of Puccinia coronata, and the wheat (10-20 plants per pot) was also inoculated by spraying with spores of Erysiphe graminis f. sp. tritici, and the cucumber (1 plant per pot) was also inoculated by spraying with spore suspension of Sphaerotheca fuliginea. These inoculated plants were further kept in a glasshouse for 10-14 days until assessment. For damping-off, the treated seeds were sown in paper pots (diameter : 7.5 cm ; depth : 6 cm) which contained soil infested with Rhizoctonia solani by mixing sterilized soil and pre-cultured fungus. Then the pots were kept in a humid glasshouse at 25-32°C.

Assessment

Damping-off was observed 15 days after sowing, and disease degree and control effect were obtained from the following equations.

$$\text{Disease Degree (\%)} = \frac{\text{Number of diseased seedlings}}{\text{Number of seeds sown}} \times 100$$

$$\text{Control (\%)} = \left(1 - \frac{\text{Disease degree in treated plot}}{\text{Disease degree in untreated plot}}\right) \times 100$$

where diseased seedlings included damping-off and unemerged seeds due to infection. For the foliar diseases, disease development was observed 10-14 days after inoculation of spores, and control effect was obtained by the following index.

| Disease Degree | Control Index |
|---|---|
| Leaves without symptom | 100 |
| Leaves covered with symptom less than 3 % area | 97 |
| Leaves covered with symptom between 4 % and 15 % area | 90 |
| Leaves covered with symptom between 16 % and 45 % area | 70 |
| Leaves covered with symptom between 46 % and 65 % area | 50 |
| Leaves covered with symptom more than 66 % | 0 |

The results are shown in Table 7.

Results

Table 7

| Compd. No. | Concentration (ppm) | Control Index | | | |
|---|---|---|---|---|---|
| | | Crown Rust in Oats | Powdery Mildew in Wheat | Rhizoctonia Damping-Off in Cucumber | Powdery Mildew in Cucumber |
| Ia - 1 | 500 | 90 | 100 | 90 | 70 |
| | 125 | 50 | 70 | 71 | 0 |
| Ia - 2 | 500 | 100 | 100 | 100 | 90 |
| | 125 | 70 | 100 | 93 | 70 |
| Ia - 3 | 500 | 100 | 100 | 94 | 70 |
| | 125 | 97 | 70 | 100 | 50 |
| Ia - 4 | 500 | 97 | 100 | 86 | 70 |
| | 125 | 50 | 50 | 53 | 50 |
| Ia - 5 | 500 | 97 | 100 | 94 | 70 |
| | 125 | 50 | 70 | 66 | 0 |
| Ia - 6 | 500 | - | - | 85 | - |
| | 125 | - | - | 54 | - |
| Ia - 7 | 500 | 100 | 70 | 94 | - |
| | 125 | 70 | 70 | 92 | - |
| Ia - 8 | 500 | - | - | 94 | - |
| | 125 | - | - | 74 | - |
| Ib - 1 | 500 | - | - | 88 | - |
| | 125 | - | - | 35 | - |
| Ib - 2 | 500 | - | - | 85 | - |
| | 125 | - | - | 60 | - |
| Triadimefon | 500 | 97 | 100 | 49 | 70 |
| | 125 | 50 | 90 | 34 | 0 |

Experiment 4

Control of Cucumber Powdery Mildew by Foliar Spray

Methods

Application of test Compound :

The test compound was dissolved in a small amount of acetone and suspended in demineralized distilled water containing a spreader, to a concentration of 7.8 ppm and 2.0 ppm. Young cucumber plants (c.v. Tsukuba-Shiroibo) of 2-leaf stage grown in pots were sprayed sufficiently with the suspension.

Inoculation of Pathogens :

Spores of Sphaerotheca fuliginea cultured in cucumber plants were collected and suspended in distilled water. Inoculation was made by spraying with the spore suspension to the test plant of 24 hr. after application of the test compound (preventive control) or 24 hr. before application (curative control).

Assessment :

Development of the disease was observed 10 days after inoculation and control index was obtained as described in Experiment 3.

The results are presented in Table 8.

Results

Table 8

| Compd. No. | Concentration (ppm) | Control Index | |
|---|---|---|---|
| | | Preventive Effect | Curative Effect |
| I a - 2 | 7.8 | 100 | 100 |
| | 2.0 | 97 | 100 |
| I a - 3 | 7.8 | 100 | 100 |
| | 2.0 | 100 | 100 |
| I a - 4 | 7.8 | 100 | 100 |
| | 2.0 | 97 | 100 |
| I a - 5 | 7.8 | 97 | 100 |
| | 2.0 | 70 | 97 |
| I a - 7 | 7.8 | 100 | 100 |
| | 2.0 | 90 | 97 |
| I a - 8 | 7.8 | 97 | 100 |
| | 2.0 | 90 | 90 |
| I b - 1 | 7.8 | 97 | 100 |
| | 2.0 | 70 | 97 |
| Triadimefon | 7.8 | 90 | 90 |
| | 2.0 | 90 | 90 |

Experiment 5

Control of Cucumber Damping-Off by Soil Drench

Methods

Application of Test Compound :

The test compound was dissolved in a small amount of acetone and suspended in demineralized distilled water containing a spreader, to a concentration of 125 ppm and 31 ppm. The soil sterilized by an autoclave was put into paper cups (diameter : 7.5 cm ; depth : 6 cm) and cucumber seeds (c.v. : Matsukaze) were sown in the cups (10 seeds/pot). Then the soil surface was covered with the following soil containing pathogen. And then 30 ml of the formation of the test compound was applied to the soil in the cup.

Inoculation of Pathogen :

Rhizoctonia solani incubated in a bran medium at 28°C for 5 days was put into sterile soil and cultured for 24 hr. The inoculated soil was diluted with sterile soil to about 4 times volume. The soil surface in the cups containing cucumber seeds was covered with the soil containing Rhizoctonia solani.

Assessment :

Control effect was judged 15 days after inoculation and control percent was obtained as described in Experiment 3.

The results are shown in Table 9.

**Results**

**Table 9**

| Compd. No. | Concentration (ppm) | Control (%) |
|---|---|---|
| I a - 1 | 125 | 100 |
| | 31 | 97 |
| I a - 2 | 125 | 100 |
| | 31 | 100 |
| I a - 3 | 125 | 100 |
| | 31 | 100 |
| I a - 4 | 125 | 100 |
| | 31 | 100 |
| I a - 5 | 125 | 100 |
| | 31 | 91 |
| I a - 7 | 125 | 100 |
| | 31 | 95 |
| I a - 8 | 125 | 100 |
| | 31 | 94 |
| Mepro-nil | 125 | 97 |
| | 31 | 80 |

## Claims

### Claims for the Contracting States : BE, CH, DE, FR, GB, IT, LI, LU, NL, SE

1. A compound of the formula :

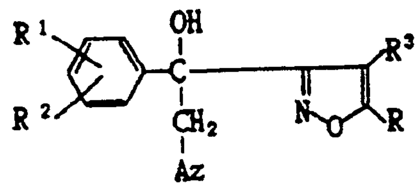

(wherein Az is imidazolyl or triazolyl ;
R is hydrogen or $C_1$-$C_5$ alkyl ;
$R^1$, $R^2$ and $R^3$ are each independently hydrogen or halogen), the compound optionally being as a salt thereof.

2. 1-(4-chlorophenyl)-1-(isoxazol-3-yl)-2-(1H-1,2,4-triazol-1-yl)ethanol.

3. An agricultural fungicidal formulation comprising a fungicidal compound according to claim 1 or claim 2 formulated for agricultural use.

4. A formulation according to claim 3, which further comprises one or more agriculturally acceptable carriers, and optionally one or more adjuvants and/or one or more other agricultural chemicals.

5. The use of a compound according to claim 1 or claim 2 or a formulation according to claim 3 or claim 4 as a fungicide other than for the treatment of the human or animal body by therapy, or a method of controlling fungus (other than a method of treatment of the human or animal body by therapy) comprising applying to the fungus or its environment a compound according to claim 1 or claim 2 or a formulation according to claim 3 or claim 4.

6. A pharmaceutical or veterinary antimycotic formulation comprising an antimycotic compound according to claim 1 or claim 2 formulated for pharmaceutical or veterinary use, respectively.

7. A pharmaceutical or veterinary formulation according to claim 6, which further comprises a pharmaceutically or veterinarily acceptable carrier, diluent or excipient and/or is in unit dosage form.

8. A compound according to claim 1 or claim 2 or a formulation according to claim 6 or claim 7 for use as a pharmaceutical or a veterinary medicament.

9. A process for preparing a compound according to claim 1 which comprises reacting a compound of the formula :

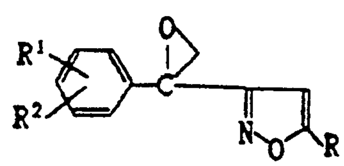
(IV)

(wherein R, $R^1$ and $R^2$ are each as defined in claim 1) with an alkali metal salt of an azole in a solvent to give a compound of the formula :

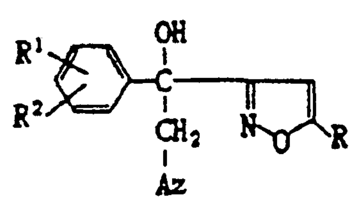
(Ia)

(wherein R, $R^1$, $R^2$ and Az are as defined in claim 1) and, if necessary, subjecting said product (Ia) to haloge-

nation to give a compound of the formula :

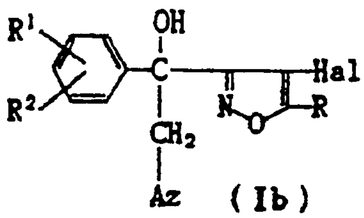

( Ib )

10. A process as claimed in claim 9, wherein the compound IV is obtained by subjecting a compound of the formula :

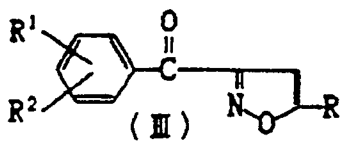

( III )

to oxirane formation by contacting it with trimethylsulfoxonium iodide in the presence of a metal hydride or in the presence of a phase transfer catalyst, the compound of Formula III optionally being obtained by reacting a compound of the formula :

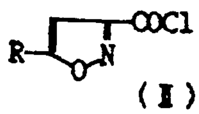

( II )

with a compound of the formula :

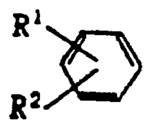

( V )

in the presence of a Lewis acid.

**Claims for the Contracting State : GR**

1. A compound of the formula :

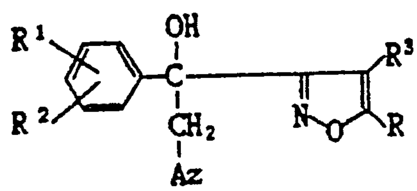

(wherein Az is imidazolyl or triazolyl ;

R is hydrogen or $C_1$-$C_5$ alkyl ;

$R^1$, $R^2$ and $R^3$ are each independently hydrogen or halogen), the compound optionally being as a salt thereof and not being for use as a pharmaceutical.

2. 1-(4-chlorophenyl)-1-(isoxazol-3-yl)-2-(1H-1,2,4-triazol-1-yl)ethanol other than for use as a pharmaceutical.

3. An agricultural fungicidal formulation comprising a fungicidal compound according to claim 1 or claim

2 formulated for agricultural use.

4. A formulation according to claim 3, which further comprises one or more agriculturally acceptable carriers, and optionally one or more adjuvants and/or one or more other agricultural chemicals.

5. The use of a compound according to claim 1 or claim 2 or a formulation according to claim 3 or claim 4 as a fungicide other than for the treatment of the human or animal body by therapy.

6. A method of controlling fungus (other than a method of treatment of the human or animal body by therapy) comprising applying to the fungus or its environment a compound according to claim 1 or claim 2 or a formulation according to claim 3 or claim 4.

7. A use according to claim 5 which is use as an agricultural fungicide or a fungicidal coating additive, or a method according to claim 6 which comprises applying the compound or formulation to soil or to a plant or its environment, or applying a coating containing the compound to a substrate.

8. A process for preparing a compound according to claim 1 which comprises reacting a compound of the formula :

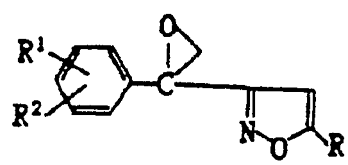

(IV)

(wherein R, $R^1$ and $R^2$ are each as defined in claim 1) with an alkali metal salt of an azole in a solvent to give a compound of the formula :

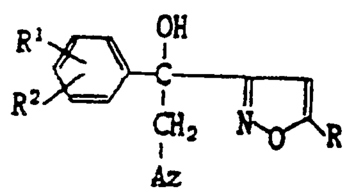

(Ia)

(wherein R, $R^1$, $R^2$ and Az are each as defined in claim 1) and, if necessary, subjecting said product (Ia) to halogenation to give a compound of the formula :

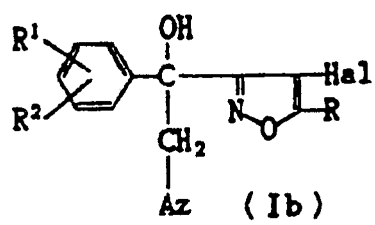

(Ib)

9. A process as claimed in claim 8 wherein the compound IV is obtained by subjecting a compound of the formula :

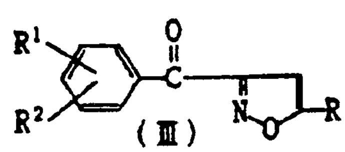

(III)

to oxirane formation by contacting it with trimethylsulfoxonium iodide in the presence of a metal hydride or in the presence of a phase transfer catalyst, the compound of formula III optionally being obtained by reacting a compound of the formula :

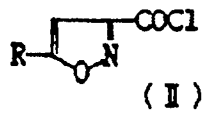

( II )

with a compound of the formula :

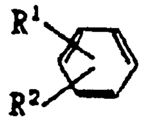

( V )

in the presence of a Lewis acid.

10. A method of preparing a pharmaceutical or veterinary formulation, comprising performing a process according to claim 8 or claim 9 to prepare a compound of formula IV and formulating the compound for pharmaceutical or veterinary use.

**Claims for the Contracting States : AT, ES**

1. A method of controlling fungus (other than a method of treatment of the human or animal body by therapy), comprising applying to the fungus or its environment a fungicidal compound of the formula :

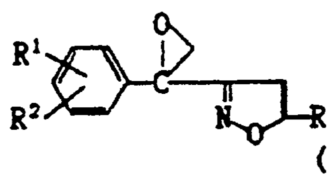

( IV )

wherein Az is imidazolyl or triazolyl ;

R is hydrogen or $C_1$-$C_5$ alkyl ; and

$R^1$, $R^2$ and $R^3$ are each independently hydrogen or halogen, the compound optionally being as a salt thereof.

2. A method according to claim 1 which comprises applying the compound to soil or to a plant or its environment, or applying a coating containing the compound to a substrate.

3. The use of a compound as defined in claim 1 as a fungicide other than for the treatment of the human or animal body by therapy.

4. A use according to claim 3 which is use as an agricultural fungicide or a fungicidal coating additive.

5. A process for preparing a compound of the formula defined in claim 1 which comprises reacting a compound of the formula :

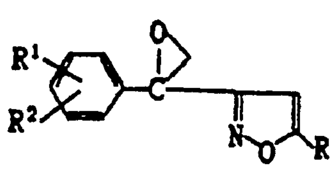

( IV )

(wherein R, $R^1$ and $R^2$ are each as defined in claim 1) with an alkali metal salt of an azole in a solvent to give a compound the formula :

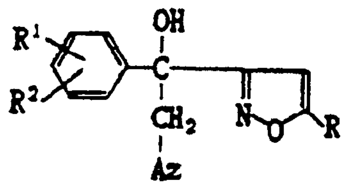 (Ia)

(wherein R, $R^1$, $R^2$ and Az are each as defined in claim 1) and, if necessary, subjecting said product (Ia) to halogenation to give a compound of the formula :

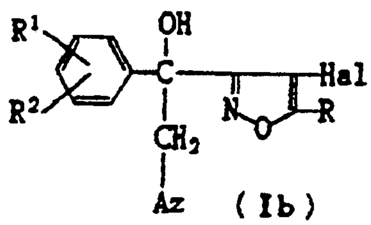

(Ib)

6. A process according to claim 5 which is carried out at a temperature of from 20 to 100°C.

7. A process according to claim 5 or claim 6, wherein the solvent is dimethylformamide.

8. A process as claimed in any one of claims 5 to 7, wherein the compound IV is obtained by subjecting a compound of the formula :

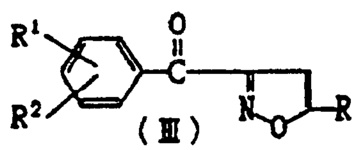

(III)

to oxirane formation by contacting it with trimethylsulfoxonium iodide in the presence of a metal hydride or in the presence of a phase transfer catalyst, the compound of formula III optionally being obtained by reacting a compound of the formula :

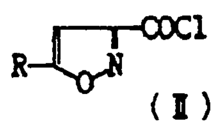

(II)

with a compound of the formula :

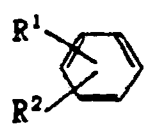 (V)

in the presence of a Lewis acid.

9. A method of preparing a pharmaceutical or veterinary formulation, comprising performing a process according to any one of claims 5 to 8 to prepare a compound of Formula IV and formulating the compound for pharmaceutical or veterinary use.

10. A method, use or process according to any one of the preceding claims, wherein the compound defined in claim 1 is 1-(4-chlorophenyl)-1-(isoxazol-3-yl)-2-(1H-1,2,4-triazol-1-yl)ethanol.

## Ansprüche

**Patentansprüche für die Vertragsstaaten : BE, CH, DE, FR, GB, IT, LI, LU, NL, SE**

1. Verbindung der Formel ;

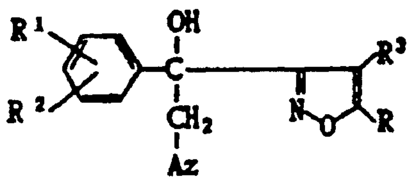

(Az ist Imidazolyl oder Triazolyl ; R ist Wasserstoff oder $C_1$-$C_5$-Alkyl ; $R^1$, $R^2$ und $R^3$ sind jeweils unabhängig voneinander Wasserstoff oder Halogen), die Verbindung kann wahlweise als Salz vorliegen.

2. 1-(4-Chlorphenyl)-1-(isoxazol-3-yl)-2-(1H-1,2,4-triazol-1-yl)ethanol.

3. In der Landwirtschaft einsetzbare Fungizid-Formulierung, enthaltend die fungizide Verbindung nach Anspruch 1 oder Anspruch 2 in einer zur landwirtschaftlichen Verwendung geeigneten Formulierung.

4. Rezeptur nach Anspruch 3, die als weitere Bestandteile einen oder mehr in der Landwirtschaft verwendbaren Trägerstoff(e) und wahlweise einen oder mehrere Hilfsstoff(e) und/oder einen oder mehrere andere in der Landwirtschaft einsetzbare chemische Stoffe enthält.

5. Verwendung einer Verbindung nach Anspruch 1 oder 2 oder einer Rezeptur nach Anspruch 3 oder 4 als Fungizid, jedoch nicht zur therapeutischen Behandlung beim Menschen oder Tier, oder eine Methode zur Kontrolle von Pilzen (jedoch nicht zu einer Methode zur therapeutischen Behandlung beim Menschen oder Tier), dadurch gekennzeichnet, daß der Pilz oder seine Umgebung mit einer Verbindung nach Anspruch 1 oder 2 oder einer Formulierung nach Anspruch 3 oder 4 behandelt wird.

6. Pharmazeutische oder veterinärmedizinische antimykotische Formulierung, enthaltend eine antimykotische Verbindung nach Anspruch 1 oder 2, entsprechend zur pharmazeutischen oder veterinärmedizinischen Verwendung formuliert.

7. Pharmazeutische oder verterinärmedizinische Formulierung nach Anspruch 6, die als weitere Bestandteile einen pharmazeutisch oder veterinärmedizinisch verwendbaren Trägerstoff, Streckmittel oder Bindemittel enthält und/oder in Form einer Einheitsdosierung vorliegt.

8. Verbindung nach Anspruch 1 oder 2 oder eine Rezeptur nach Anspruch 6 oder 7 zur Verwendung als pharmazeutisches oder verterinärmedizinisches Medikament.

9. Verfahren zur Herstellung einer Verbindung nach Anspruch 1, dadurch gekennzeichnet, daß eine Verbindung der Formel (IV)

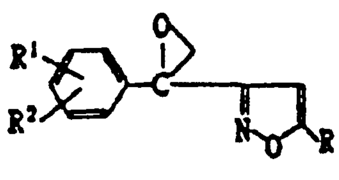
(IV)

(R, $R^1$ und $R^2$ haben dieselbe Bedeutung wie in Anspruch 1) mit einem Alkalimetallsalz eines Azols in einem Lösungsmittel unter Erhalt einer Verbindung der Formel (Ia) umgesetzt wird

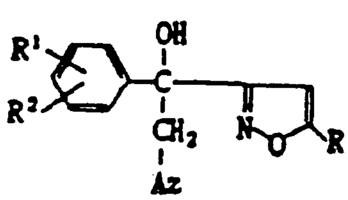
(Ia)

(R, $R^1$, $R^2$ und Az haben dieselbe Bedeutung wie in Anspruch 1), und, falls notwendig, das Produkt (Ia) unter Erhalt einer Verbindung der Formel (Ib) halogeniert wird :

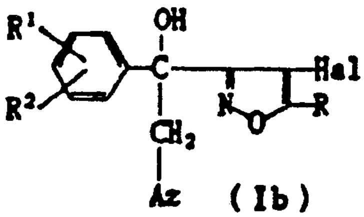

(Ib)

10. Verfahren nach Anspruch 9, dadurch gekennzeichnet, daß die Verbindung (IV) zu einem Oxiran mit der Formel (III)

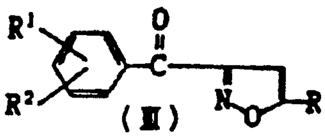

(III)

umgesetzt wird, indem Trimethylsulfoniumoxid in Gegenwart eines Metallhydrids oder in Gegenwart eines Phasentransferkatalysators mit der Verbindung (III) zusammengebracht wird ; die Verbindung (III) kann wahlweise durch Reaktion einer Verbindung der Formel (II)

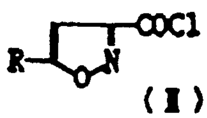

(II)

mit einer Verbindung der Formel (V)

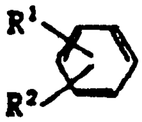

(V)

in Gegenwart einer Lewissäure erhalten werden.

**Patentansprüche für den Vertragsstaat : GR**

1. Verbindung der Formel

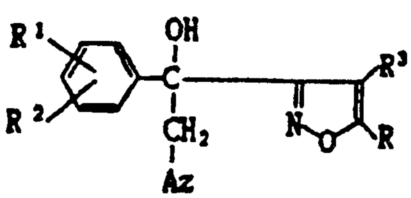

(Az ist Imidazolyl oder Triazolyl ; R ist Wasserstoff oder $C_1$-$C_5$-Alkyl, $R^1$, $R^2$ und $R^3$ sind jeweils unabhängig voneinander Wasserstoff oder Halogen), die Verbindung kann wahlweise als Salz vorliegen und dient nicht zur Verwendung als pharmazeutisches Mittel.

2. 1-(4-Chlorphenyl)-1-(isoxazol-3-yl)-2-(1H-1,2,4-triazol-1-yl)ethanol, jedoch nicht zur Verwendung als pharmazeutisches Mittel.

3. In der Landwirtschaft einsetzbare Fungizid-Formulierung, enthaltend eine fungizide Verbindung nach

Anspruch 1 oder 2, die zur landwirtschaftlichen Verwendung formuliert wurde.

4. Formulierung nach Anspruch 3, die weiterhin einen oder mehrere in der Landwirtschaft verwendbaren Trägerstoff(e) und wahlweise einen oder mehrere Hilfsstoff(e) und/oder einen oder mehrere in der Landwirtschaft verwendbare chemische Stoffe enthält.

5. Verwendung der Verbindung nach Anspruch 1 oder Anspruch 2 oder Formulierung nach Anspruch 3 oder 4 als Fungizid, jedoch nicht zur therapeutischen Behandlung am Menschen oder Tier.

6. Methode zur Kontrolle von Pilzen (jedoch nicht therapeutische Methode zur Behandlung am Menschen oder Tier), dadurch gekennzeichnet, daß der Pilz oder seine Umgebung mit einer Verbindung nach Anspruch 1 oder 2 oder einer Formulierung nach Anspruch 3 oder 4 behandelt wird.

7. Verwendung nach Anspruch 5 als landwirtschaftliches Fungizid oder fungizides Beschichtungsadditiv oder Methode nach Anspruch 6, dadurch gekennzeichnet, daß die Verbindung oder Formulierung auf die Pflanze oder ihre Umgebung aufgebracht wird oder eine Beschichtung, welche die Verbindung enthält, dem Substrat verabreicht wird.

8. Verfahren zur Herstellung einer Verbindung nach Anspruch 1, dadurch gekennzeichnet, daß eine Verbindung der Formel (IV)

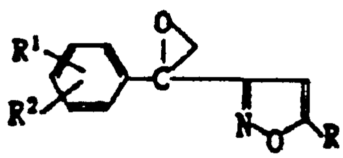

(IV)

(worin R, $R^1$ und $R^2$ dieselben Bedeutungen wie in Anspruch 1 besitzen), mit einem Alkalimetallsalz eines Azols in einem Lösungsmittel unter Erhalt einer Verbindung der Formel (Ia)

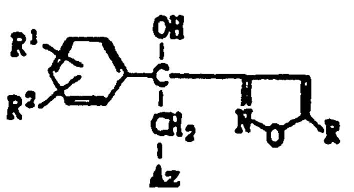

(Ia)

(R, $R^1$, $R^2$ und Az haben dieselbe Bedeutung wie in Anspruch 1) umgesetzt wird, und, falls notwendig, das Produkt (Ia) unter Erhalt der Verbindung der Formel (Ib) halogeniert wird :

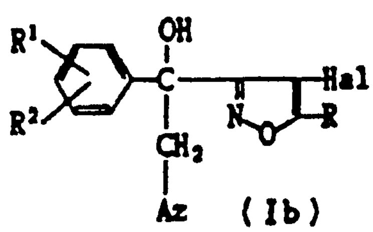

(Ib)

9. Verfahren nach Anspruch 8, dadurch gekennzeichnet, daß die Verbindung (IV) erhalten wird durch Oxiranbildung einer Verbindung der Formel (III)

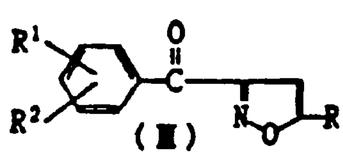

(III)

indem sie mit Trimethylsulfoxoniumjodid in Gegenwart eines Metallhydrids oder in Gegenwart eines Phasentransferkatalysators zusammengebracht wird, die Verbindung der Formel (III) kann wahlweise durch

Reaktion einer Verbindung der Formel (II)

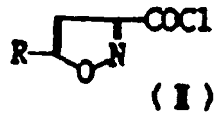

( II )

mit einer Verbindung der Formel (V)

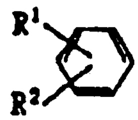

(V)

in Gegenwart einer Lewissäure erhalten werden.

10. Verfahren zur Herstellung einer pharmazeutischen oder veterinärmedizinischen Formulierung, dadurch gekennzeichnet, daß ein Verfahren nach Anspruch 8 oder 9 zur Herstellung einer Verbindung der Formel (IV) durchgeführt wird und die Verbindung zur pharmazeutischen oder verterinärmedizinischen Verwendung formuliert wird.

**Patetansprüche für die Vertragsstaaten : AT, ES**

1. Methode zur Kontrolle von Pilzen (jedoch nicht therapeutische Methode zur Behandlung am Menschen oder Tier), dadurch gekennzeichnet, daß der Pilz oder seine Umgebung mit einer fungiziden Verbindung der Formel (IV) behandelt wird :

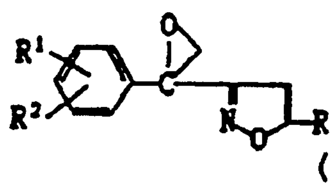

(IV)

(Az ist Imidazolyl oder Triazolyl, R ist Wasserstoff oder $C_1$-$C_5$-Alkyl ; $R^1$, $R^2$ und $R^3$ sind jeweils unabhängig voneinander Wasserstoff oder Halogen), die Verbindung kann wahlweise als Salz vorliegen.

2. Methode nach Anspruch 1, dadurch gekennzeichnet, daß die Verbindung in den Boden oder auf die Pflanze oder ihre Umgebung aufgebracht oder eine Beschichtung, die die Verbindung enthält, auf das Substrat aufgebracht wird.

3. Verwendung einer Verbindung, wie in Anspruch 1 definiert, als Fungizid, jedoch nicht zur therapeutischen Behandlung am Menschen oder Tier.

4. Verwendung nach Anspruch 3 als in der Landwirtschaft einsetzbares Fungizid oder fungizides Beschichtungsadditiv.

5. Verfahren zur Herstellung einer Verbindung der Formel, wie in Anspruch 1 definiert, dadurch gekennzeichnet, daß eine Verbindung der Formel (IV)

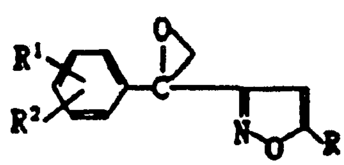

(IV)

(R, $R^1$ und $R^2$ sind wie in Anspruch 1 definiert) mit einem Alkalimetallsalz eines Azols in einem Lösungsmittel unter Erhalt einer Verbindung der Formel (Ia)

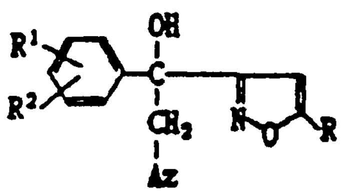

(Ia)

(R, $R^1$, $R^2$ und Az haben dieselbe Bedeutung wie in Anspruch 1) umgesetzt wird und, falls notwendig, das Produkt (Ia) unter Erhalt einer Verbindung der Formel (Ib) halogeniert wird :

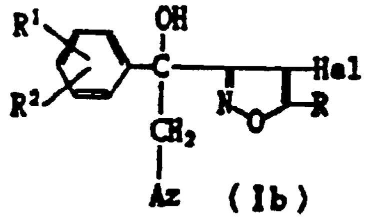

(Ib)

6. Verfahren nach Anspuch 5, dadurch gekennzeichnet, daß die Reaktionstemperatur 20 bis 100°C beträgt.

7. Verfahren nach Anspruch 5 oder 6, dadurch gekennzeichnet, daß das Lösungsmittel Dimethylformamid ist.

8. Verfahren nach einem der Ansprüche 5 bis 7, dadurch gekennzeichnet, daß die Verbindung (IV) erhalten wird durch Oxiranbildung einer Verbindung der Formel (III)

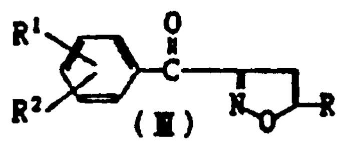

(III)

indem sie mit Trimethylsulfoxoniumjodid in Gegenwart eines Metallhydrids oder in Gegenwart eines Phasentransferkatalysators zusammengebracht wird ; die Verbindung der Formel (III) kann wahlweise durch Reaktion einer Verbindung der Formel (II)

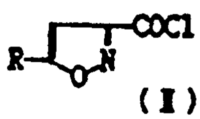

(II)

mit einer Verbindung der Formel (V)

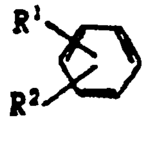

(V)

in Gegenwart einer Lewissäure erhalten werden.

9. Verfahren zur Herstellung einer pharmazeutischen oder veterinärmedizinischen Formulierung, dadurch gekennzeichnet, daß ein Verfahren nach einem der Ansprüche 5 bis 8 durchgeführt wird, wobei eine Verbindung der Formel (IV) hergestellt wird und diese Verbindung zur pharmazeutischen oder veterinärmedizinischen Verwendung formuliert wird.

10. Verfahren oder Verwendung nach einem oder mehreren der vorhergehenden Ansprüche, dadurch gekennzeichnet, daß die Verbindung wie in Anspruch 1 definiert 1-(4-Chlorphenyl)-1-(isoxazol-3-yl)-2-(1H-1,2,4-triazol-1-yl)ethanol ist.

**Revendications**

**Revendications pour les Etats contractants : BE, CH, DE, FR, GB, IT, LI, LU, NL, S**

1. Composé répondant à la formule :

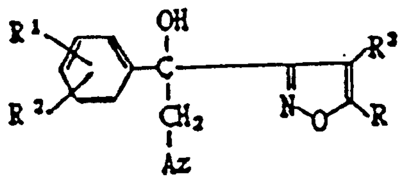

(dans laquelle Az représente l'imidazolyle ou le triazolyle ; R représente l'hydrogène ou un $C_1$-$C_5$-alkyle ; $R^1$, $R^2$ et $R^3$ représentent chacun, de manière indépendante, l'hydrogène ou un halogène), le composé se présentant éventuellement sous la forme d'un de ses sels.

2. 1-(4-chlorophényl)-1-(isoxazol-3-yl)-2-(1H-1,2,4-triazol-1-yl)éthanol.

3. Composition fongicide agricole, caractérisée en ce qu'elle comprend un composé fongicide selon la revendication 1 ou 2, formulée pour une mise en oeuvre en agriculture.

4. Composition selon la revendication 3, caractérisée en ce qu'elle comprend en outre un ou plusieurs supports acceptables du point de vue agricole, et éventuellement un ou plusieurs adjuvants et/ou un ou plusieurs autres produits chimiques agricoles.

5. Mise en oeuvre du composé selon la revendication 1 ou 2 ou d'une composition selon la revendication 3 ou 4 comme fongicide autre que pour le traitement du corps humain ou animal par thérapie, ou d'un procédé pour maîtriser les champignons (autre qu'un procédé de traitement du corps humain ou animal par thérapie), caractérisé en ce qu'il comprend l'application, sur le champignon ou son milieu, d'un composé selon la revendication 1 ou 2 ou d'une composition selon la revendication 3 ou 4.

6. Composition antimycotique pharmaceutique ou vétérinaire, caractérisée en ce qu'elle comprend un composé antimycotique selon la revendication 1 ou 2, formulée respectivement pour une mise en oeuvre pharmaceutique ou vétérinaire.

7. Composition pharmaceutique ou vétérinaire selon la revendication 6, caractérisée en ce qu'elle comprend en outre un support, un diluant ou un excipient acceptable du point de vue pharmaceutique ou vétérinaire, et/ou se présente sous forme de dose unitaire.

8. Composé selon la revendication 1 ou 2 ou composition selon la revendication 6 ou 7, destiné à être mis en oeuvre comme médicament pharmaceutique ou vétérinaire,

9. Procédé de préparation d'un composé selon la revendication 1, caractérisé en ce qu'on fait réagir un composé répondant à la formule :

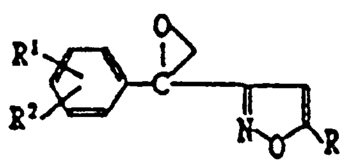

(IV)

(dans laquelle R, $R^1$ et $R^2$ sont définis chacun comme dans la revendication 1), avec un sel de métal alcalin d'un pyrrole dans un solvant pour donner un composé répondant à la formule :

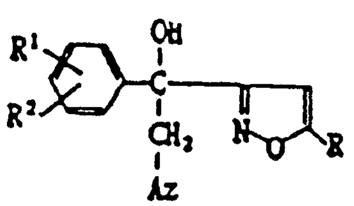

(I a)

(dans laquelle R, $R^1$, $R^2$ et Az sont définis chacun comme dans la revendication 1) et, si nécessaire, on soumet ledit produit (Ia) à une halogénation pour donner un composé répondant à la formule :

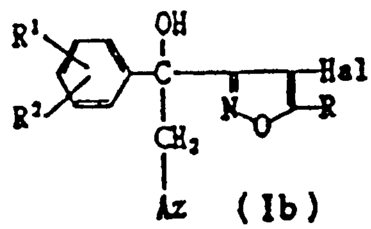

(Ib)

10. Procédé selon la revendication 9, caractérisé en ce qu'on obtient le composé IV en soumettant un composé répondant à la formule :

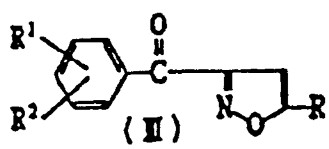

(III)

à une formation d'oxyranne en le mettant en contact avec de l'iodure de triméthylsulfoxonium en présence d'un hydrure de métal ou en présence d'un catalyseur de transfert de phase, le composé de formule III étant éventuellement obtenu par réaction d'un composé répondant à la formule :

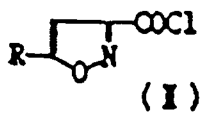

(II)

avec un composé répondant à la formule :

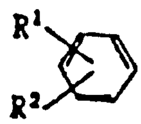

(V)

en présence d'un acide de Lewis.

**Revendications pour l'Etat contractant : GR**

1. Composé répondant à la formule :

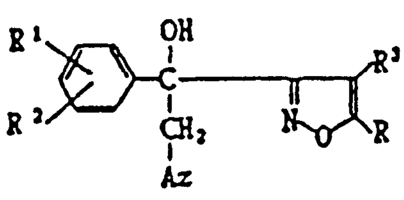

(dans laquelle Az représente l'imidazolyle ou le triazolyle ; R représente l'hydrogène ou un $C_1$-$C_5$-alkyle ; $R^1$, $R^2$ et $R^3$ représentent chacun, de manière indépendante, l'hydrogène ou un halogène), le composé se présentant éventuellement sous la forme d'un de ses sels et n'étant pas destiné pour être mis en oeuvre comme produit pharmaceutique.

2. 1-(4-chlorophényl)-1-(isoxazol-3-yl)-2-(1H-1,2,4-triazol-1-yl)éthanol, non destiné à être mis en oeuvre comme produit pharmaceutique.

3. Composition fongicide agricole, caractérisée en ce qu'elle comprend un composé fongicide selon la revendication 1 ou 2, formulée pour une mise en oeuvre en agriculture.

4. Composition selon la revendication 3, caractérisée en ce qu'elle comprend en outre un ou plusieurs supports acceptables du point de vue agricole, et éventuellement un ou plusieurs adjuvants et/ou un ou

plusieurs autres produits chimiques agricoles.

5. Mise en oeuvre du composé selon la revendication 1 ou 2 ou d'une composition selon la revendication 3 ou 4 comme fongicide autre que pour le traitement du corps humain ou animal par thérapie.

6. Procédé pour maîtriser les champignons (autre qu'un procédé de traitement du corps humain ou animal par thérapie), caractérisé en ce qu'il comprend l'application, sur le champignon ou son milieu, d'un composé selon la revendication 1 ou 2 ou d'une composition selon la revendication 3 ou 4.

7. Mise en oeuvre selon la revendication 5, en tant que fongicide agricole ou qu'additif d'enduit fongicide, ou procédé selon la revendication 6, caractérisé en ce qu'on applique le composé ou la composition sur un sol ou sur une plante ou son milieu, ou on applique un enduit, contenant le composé, sur un substrat.

8. Procédé de préparation d'un composé selon la revendication 1, caractérisé en ce qu'on fait réagir un composé répondant à la formule :

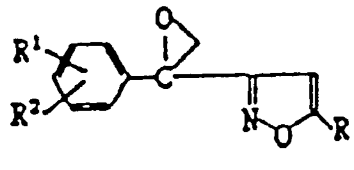

(IV)

(dans laquelle R, $R^1$ et $R^2$ sont définis chacun comme dans la revendication 1), avec un sel de métal alcalin d'un pyrrole dans un solvant pour donner un composé répondant à la formule :

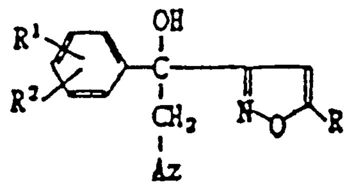

(Ia)

(dans laquelle R, $R^1$, $R^2$ et Az sont définis chacun comme dans la revendication 1) et, si nécessaire, on soumet ledit produit (Ia) à une halogénation pour donner un composé répondant à la formule :

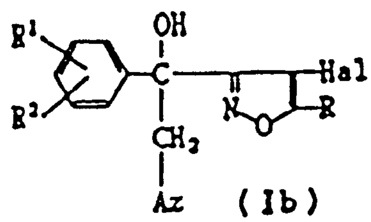

(Ib)

9. Procédé selon la revendication 8, caractérisé en ce qu'on obtient le composé IV en soumettant un composé de formule :

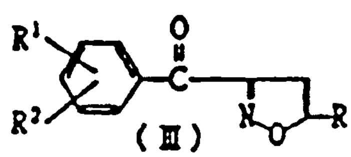

(III)

à une formation d'oxyranne en le mettant en contact avec de l'iodure de triméthylsulfoxonium en présence d'un hydrure de métal ou en présence d'un catalyseur de transfert de phase, le composé de formule III étant éventuellement obtenu par réaction d'un composé répondant à la formule :

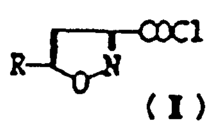

(II)

avec un composé répondant à la formule :

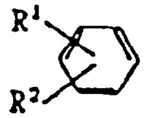

(V)

en présence d'un acide de Lewis.

10. Procédé de préparation d'une composition pharmaceutique ou vétérinaire, caractérisé en ce qu'on met en oeuvre un procédé selon la revendication 8 ou 9 pour préparer un composé de formule V et on formule ledit composé pour une mise en oeuvre pharmaceutique ou vétérinaire.

**Revendications pour les Etats contractants : AT, ES**

1. Procédé pour maîtriser les champignons (autre qu'un procédé de traitement du corps humain ou animal par thérapie), caractérisé en ce qu'on applique sur le champignon ou son milieu un composé fongicide répondant à la formule :

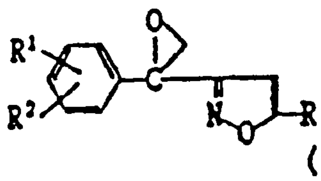

(IV)

dans laquelle Az représente l'imidazolyle ou le triazolyle ; R représente l'hydrogène ou un $C_1$-$C_5$-alkyle ; et $R^1$, $R^2$ et $R^3$ représentent chacun, de manière indépendante, l'hydrogène ou un halogène, le composé pouvant éventuellement se présenter sous la forme d'un de ses sels.

2. Procédé selon la revendication 1, caractérisé en ce qu'on applique le composé sur un sol ou sur une plante ou son milieu, ou on applique un enduit, contenant le composé, sur un substrat.

3. Mise en oeuvre du composé selon la revendication 1 comme fongicide autre que pour le traitement du corps humain ou animal par thérapie.

4. Mise en oeuvre selon la revendication 3, en tant que fongicide agricole ou qu'additif d'enduit fongicide.

5. Procédé de préparation d'un composé répondant à la formule définie dans la revendication 1, caractérisé en ce qu'on fait réagir un composé répondant à la formule :

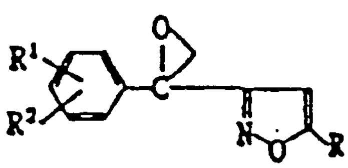

(IV)

(dans laquelle R, $R^1$ et $R^2$ sont définis chacun comme dans la revendication 1), avec un sel de métal alcalin d'un pyrrole dans un solvant pour donner un composé répondant à la formule :

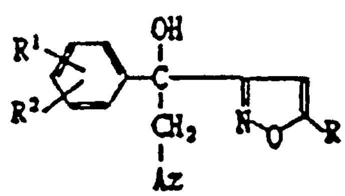

(Ia)

(dans laquelle R, $R^1$, $R^2$ et Az sont définis chacun comme dans la revendication 1) et, si nécessaire, on soumet ledit produit (Ia) à une halogénation pour donner un composé répondant à la formule :

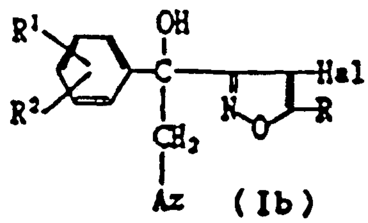

(Ib)

6. Procédé selon la revendication 5, caractérisé en ce qu'il est réalisé à une température comprise entre 20 et 100°C.

7. Procédé selon la revendication 5 ou 6, caractérisé en ce que le solvant est le diméthylformamide.

8. Procédé selon l'une quelconque des revendications 5 à 7, caractérisé en ce qu'on obtient le composé IV en soumettant un composé répondant à la formule :

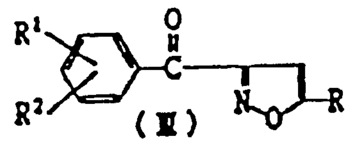

(II)

à une formation d'oxyranne en le mettant en contact avec de l'iodure de triméthylsulfoxonium, en présence d'un hydrure de métal ou en présence d'un catalyseur de transfert de phase, le composé de formule III étant éventuellement obtenu par réaction d'un composé répondant à la formule :

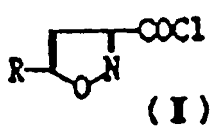

(I)

avec un composé répondant à la formule :

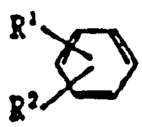

(V)

en présence d'un acide de Lewis.

9. Procédé de préparation d'une composition pharmaceutique ou vétérinaire, caractérisé en ce qu'on met en oeuvre un procédé selon l'une quelconque des revendications 5 à 8 pour préparer un composé répondant à la formule IV et on formule le composé pour une mise en oeuvre pharmaceutique ou vétérinaire.

10. Procédé ou mise en oeuvre selon l'une quelconque des revendications précédentes, caractérisé en ce que le composé défini dans la revendication 1 est le 1-(4-chlorophényl)-1-(isoxazol-3-yl)-2-(1H-1,2,4-triazol-1-yl)éthanol.